(19) **Europäisches Patentamt / European Patent Office / Office européen des brevets**

(11) **EP 4 769 422 A1**

(12) **EUROPEAN PATENT APPLICATION**

(43) Date of publication:
01.07.2026 Bulletin 2026/27

(21) Application number: 25227427.9

(22) Date of filing: 29.12.2025

(51) International Patent Classification (IPC):
$G16H\ 20/17^{(2018.01)}$    $A61M\ 5/172^{(2006.01)}$
$G16H\ 50/20^{(2018.01)}$    $G16H\ 50/70^{(2018.01)}$

(52) Cooperative Patent Classification (CPC):
G16H 20/17; A61M 5/1723; G16H 50/20;
G16H 50/70; A61M 2005/14208; A61M 2230/201

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.12.2024 CN 202411959014

(71) Applicant: Shanghai United Imaging
Microelectronics
Technology Co., Ltd.
Shanghai 201800 (CN)

(72) Inventors:
• Wang, Yunhui
Shanghai, 201800 (CN)
• Mu, Qingfeng
Shanghai, 201800 (CN)
• Liu, Hongxing
Shanghai, 201800 (CN)
• Huang, Xiaoyan
Shanghai, 201800 (CN)

(74) Representative: Petraz, Gilberto Luigi et al
GLP S.r.l.
Viale Europa Unita, 171
33100 Udine (IT)

(54) **INSULIN PUMP CONTROL METHOD AND APPARATUS, ARTIFICIAL PANCREATIC CLOSED-LOOP SYSTEM, AND STORAGE MEDIUM**

(57) An insulin pump control method and apparatus, an artificial pancreatic closed-loop system, and a storage medium are provided. The method includes: acquiring first monitoring data of a target object, obtaining an injection dosage of a first injection according to a target model and the first monitoring data, and injecting the target object based on the injection dosage of the first injection. The target model is adjusted based on total dosages corresponding to historical prediction failures.

| Acquiring first monitoring data of a target object | S310 |

| Obtaining an injection dosage of a first injection according to a target model and the first monitoring data. The target model is adjusted based total dosages corresponding to historical prediction failures | S320 |

| Injecting the target object based on the injection dosage of the first injection | S330 |

FIG. 3

**Description**

**TECHNICAL FIELD**

The present invention relates to the field of medical apparatus technology, and in particular, to an insulin pump control method and apparatus, an artificial pancreatic closed-loop system, and a storage medium.

**BACKGROUND**

Current insulin pumps typically require users to input a desired drug dosage to be injected, before driving a motor to deliver insulin through a tubing. The whole process is not only cumbersome, but also makes it difficult for even experienced diabetic patients to accurately control the insulin dosage to be injected. Insufficient or excessive injection dosage poses risks of glycemic disorders.

For the issues of inaccurate injection dosage and poor user experience of the insulin pump in the related art, no effective solution has been proposed.

**SUMMARY**

In embodiments of the present invention, an insulin pump control method and apparatus, an artificial pancreatic closed-loop system, and a storage medium are provided.

In a first aspect, an insulin pump control method is provided, including: acquiring first monitoring data of a target object; obtaining an injection dosage of a first injection according to a target model and the first monitoring data; and injecting the target object based on the injection dosage. The target model is adjusted based on total dosages corresponding to historical prediction failures.

In some embodiments, the method further includes: after the first injection is completed, continuously acquiring second monitoring data of the target object, comparing the second monitoring data with a preset threshold of blood glucose concentration, and determining whether to end the injection based on the comparison result.

In some embodiments, determining whether to end the injection based on the comparison result further includes: when the second monitoring data satisfies the preset threshold of blood glucose concentration, ending the injection.

In some embodiments, determining whether to end the injection based on the comparison result further includes: when the second monitoring data does not accord with the preset threshold of blood glucose concentration, triggering a preset protection mechanism. The protection mechanism is configured for adjusting an injection dosage to perform the injection again until the second monitoring data satisfies the preset threshold of blood glucose concentration.

In some embodiments, the target model includes extreme learning machine base models.

In some embodiments, the method further includes: acquiring a total dosage of a prediction failure; and adjusting weights of extreme learning machine base models in the target model based on the total dosage of the prediction failure.

In some embodiments, adjusting the weights of the extreme learning machine base models in the target model based on the total dosage of the prediction failure, includes: determining a first group of base models based on the total dosage of the prediction failure, improving weights of the first group of base models in the target model, and allocating remaining weights to non-first group of base models. The improved weights of the first group of base models are higher than the allocated weights of the non-first group of base models.

In some embodiments, determining the first group of base models based on the total dosage of the prediction failure, includes: when a prediction error between a predicted dosage of an extreme learning machine base model in the target model and the total dosage of the prediction failure meets a preset condition, determining that the extreme learning machine base model belongs to the first group of base models.

In some embodiments, when prediction errors between predicted dosages of at least two extreme learning machine base models and the total dosage of the prediction failure meet the preset condition, one of the at least two extreme learning machine base models is selected to the first group of base models.

The present condition includes: the prediction error is less than a preset prediction error threshold, or the prediction error is less than an error between a predicted dosage of other extreme learning machine base model and the total dosage of the prediction failure.

In some embodiments, the weights of the non-first group of base models are same or different.

In some embodiments, the first monitoring data includes a body temperature.

In some embodiments, the method further includes: pre-training the target model. Pre-training the target model includes: dividing an acquired dataset into a training set and a verification set; performing iterative fusion training on a preset first regression model based on the training set to obtain extreme learning machine base models; inputting the verification set into the extreme learning machine base models to obtain corresponding characteristic values; and performing reinforcement training on a preset second regression model according to label values and the characteristic values of the verification set to obtain an extreme learning machine meta model, completing model training, and determining the target model. The dataset includes changes in blood glucose concentration caused by dosages of different individuals, and the training set has the same quantity of samples in different days. A first layer of the target model includes the extreme learning machine base models, a second layer of the target model includes the extreme learning machine meta model, and the extreme learning machine meta model is configured to output a predicted dosage.

In some embodiments, performing the iterative fusion training on the preset first regression model based on the training set to obtain the extreme learning machine base models, includes: initializing weights of the samples in the training set based on preset number of iterations, and determining a sampling probability of the samples; selecting first training samples for a current iteration round from the training set based on the sampling probability; performing fusion training on the preset first regression model based on the first training samples to obtain an extreme learning machine base model for the current iteration round; and when error rates corresponding to the characteristic values obtained in all iteration rounds meet a termination condition, obtaining all the extreme learning machine base models.

In some embodiments, performing the reinforcement training on the preset second regression model according to the label values and the characteristic values of the verification set to obtain the extreme learning machine meta model, includes: constructing second training samples for the preset second regression model according to the label values and the characteristic values of the verification set; and performing the reinforcement training on the second regression model based on the second training samples to obtain the extreme learning machine meta model.

In a second aspect, another insulin pump control method is provided, including: acquiring first monitoring data of a target object, inputting the first monitoring data into a trained extreme learning machine model to determine an injection dosage, and injecting the target object based on the injection dosage. The first monitoring data includes blood glucose data.

In some embodiments, the trained extreme learning machine model includes an extreme learning machine base model and an extreme learning machine meta model, an input of the extreme learning machine base model includes the first monitoring data, an output of the extreme learning machine base model is taken as an input of the extreme learning machine meta model, and an output of the extreme learning machine meta model includes the injection dosage.

In some embodiments, the method further includes: after the injection is completed, continuously acquiring second monitoring data of the target object, and comparing the second monitoring data with a preset threshold of blood glucose concentration, and when the second monitoring data does not accord with the preset threshold of blood glucose concentration, performing injection again according to a preset dosage.

In some embodiments, the method further includes: determining, based on a combination of the injection dosage and the reinjection dosage, whether a difference between the output of the extreme learning machine base model and the combination meets a preset condition; if the difference between the output of the extreme learning machine base model and the combination meets the preset condition, adjusting a weight of the extreme learning machine base model in the trained extreme learning machine model.

In a third aspect, an insulin pump control apparatus is provided, which includes an acquisition module, a processing module, and an injecting module. The acquisition module is configured to acquire first monitoring data of a target object. The processing module is configured to obtain an injection dosage of a first injection according to a target model and the first monitoring data. The target model is adjusted based on total dosages corresponding to historical prediction failures. The injecting module is configured to inject the target object based on the injection dosage of the first injection.

In a fourth aspect, an artificial pancreas closed-loop system is provided, which includes a memory, a processor, and a computer program stored in the memory and executable by the processor. The processor is configured to implement the insulin pump control method described in the first aspect when executing the computer program.

In a fifth aspect, a storage medium having a computer program stored thereon is provided. The computer program is configured to implement the insulin pump control method described in the first aspect when executed by a processor.

Details of one or more embodiments of the present invention are set forth in the accompanying drawings and description below to make other features, objects, and advantages of the present invention clearer and more comprehensible.

## BRIEF DESCRIPTION OF THE DRAWINGS

The accompanying drawings described herein are provided to further illustrate the understanding of the present invention and constitute a part of the present invention. The illustrative embodiments and descriptions of the present invention are intended to explain the present invention, and do not constitute an improper limitation on the present invention.

FIG. 1 is a hardware structural block diagram of an artificial pancreas closed-loop system in an embodiment of the present invention.

FIG. 2 is a hardware structural block diagram of a lower-level computer in an embodiment of the present invention.

FIG. 3 is a flowchart of an insulin pump control method in an embodiment of the present invention.

FIG. 4 is a flowchart of a protection mechanism operation in an embodiment of the present invention.

FIG. 5 is a flowchart of target model training in an embodiment of the present invention.

FIG. 6 is a schematic flowchart of model training in an embodiment of the present invention.

FIG. 7 is a structural block diagram of an insulin pump control apparatus in an embodiment of the present invention.

In the drawings: 10 represents a host computer; 20 represents a lower-level computer; 21 represents a control unit; 22 represents a transmitter; 23 represents an insulin pump; 24 represents a temperature sensor; 25 represents a power management unit; 210 represents an acquiring module; 220 represents a processing module; and 230 represents an injecting module.

## DETAILED DESCRIPTION

To more clearly understand objects, technical solutions, and advantages of the present invention, the present invention is illustrated and described below with reference to the accompanying drawings and embodiments.

Unless otherwise defined, technical or scientific terms used in the present invention shall have a general meaning as understood by those skilled in the art to which the present invention belongs. The terms such as "a", "an", "the", "this", and "these" in the present invention do not denote a quantitative limitation and may be singular or plural. The terms such as "comprise", "include", "have", and any variations thereof used in the present invention are intended to cover a non-exclusive inclusion. For example, a process, a method, a system, a product, or a device including a series of steps or modules (units) is not limited to listed steps or modules (units) but may include steps or modules (units) not listed or inherent to the process, the method, the product, or the device. The terms such as "connect", "link", and "couple" used in the present invention are not limited to physical or mechanical connections but may include electrical connections, whether direct or indirect connections. The term "a plurality of" as used in the present invention refers to two or more. The term "and/or" describes an associative relationship between associated objects, indicating that three relationships may exist. For example, "A and/or B" may indicate the following three cases: A exists alone, both A and B exist, or B exists alone. Generally, the character "/" indicates that the associated objects are in an "or" relationship. The terms such as "first", "second", and "third" used in the present invention are merely used to distinguish similar objects and do not denote a specific order of the objects.

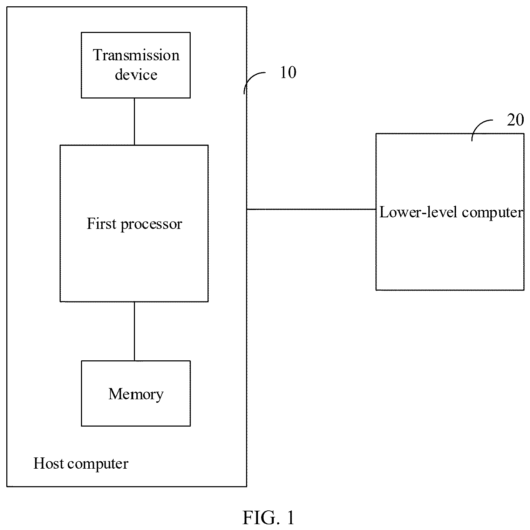

The method embodiments according to the present invention may be executed in an artificial pancreas closed-loop system, terminal, computer, or similar computing device. For example, the method may be executed in the artificial pancreas closed-loop system, which includes a memory and a processor. The memory may be configured to store computer programs, such as software programs and modules of application software, including the computer programs corresponding to all method embodiments in the present invention. The processor may be configured to perform various functional applications and data processing by executing the computer programs stored in the memory. As another example, the hardware structural block diagram of the artificial pancreas closed-loop system is shown in FIG. 1, which may include a host computer 10 and a lower-level computer 20. The host computer 10 may include a first processor and a first memory configured to store data. The first processor may include, but not be limited to, a processing device such as a Microcontroller Unit (MCU) or a Field-Programmable Gate Array (FPGA). The host computer 10 may also include a first transmission device for communication functions. Those skilled in the art may understand that the structure shown in FIG. 1 is merely illustrative and does not impose limitations on the structure of the aforementioned terminal. For example, the artificial pancreas closed-loop system may also include more or fewer components than those shown in FIG. 1 or may have a configuration different from that shown in FIG. 1.

The first memory may be configured to store computer programs, such as software programs and modules of application software, including the computer programs corresponding to relevant method embodiments (e.g., including step 510 to step 530, etc.) related to model training in this embodiment. The first processor may be configured to perform various functional applications and data processing by executing the computer programs stored in the first memory, thereby implementing the aforementioned methods related to model training.

The lower-level computer 20 may include a second processor and a second memory configured to store data. The second processor may be the same as the first processor and may also include, but not be limited to, a processing device such as a Microcontroller Unit (MCU) or a Field-Programmable Gate Array (FPGA). The lower-level computer 20 may further include a second transmission device for communication functions. The second memory may be configured to store computer programs, such as software programs and modules of application software, including the computer programs corresponding to relevant method embodiments related to insulin pump control in this embodiment (e.g., including step 310 to step 330, etc.). The second processor may be configured to execute various functional applications and data processing by executing the computer programs stored in the second memory, thereby implementing the aforementioned methods related to insulin pump control.

The first memory and the second memory may have the same hardware structure. Each of the first memory and the second memory may include a high-speed random access memory, a non-volatile memory such as one or more magnetic storage apparatus and flash memory, or other non-volatile solid-state memory. In some examples, each of the first memory and/or the second memory may further include memory remotely located relative to the first processor and/or the second processor. Such remote memory may be electrically connected to the artificial pancreas closed-loop system via a network. Examples of the network include, but are not limited to, the Internet, intranets, local area networks, mobile communication networks, and combinations thereof.

The first transmission device and the second transmission device may be configured to receive or transmit data via a network. The network may include wireless networks provided by communication suppliers of the artificial pancreas closed-loop system. In one example, each of the first transmission device and the second transmission device may include a Network Interface Controller (NIC), which can be connected to other network devices via base stations to communicate with the Internet. In another example, each of the first transmission device and the second transmission device may be a Radio Frequency (RF) module, configured to communicate with the Internet wirelessly. The first transmission device may transmit a target model trained by the host computer 10 to the lower-level computer 20. The lower-level computer 20 may receive the target model via the second transmission device, thereby completing writing of the target model.

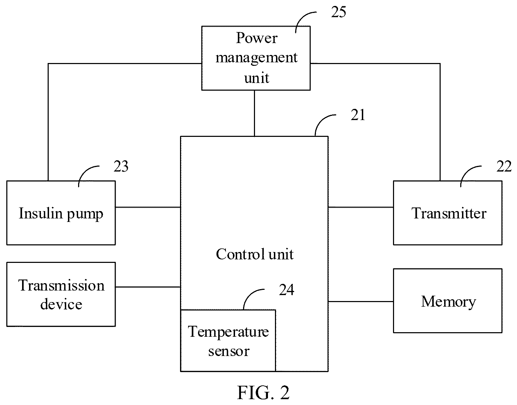

Referring to FIG. 2, the lower-level computer may be a medical device worn by a target object. The lower-level computer may further include a transmitter 22, a control unit 21, an insulin pump 23, a temperature sensor 24, and a power management unit 25. The power management unit 25 may be connected to the transmitter 22, the control unit 21, and the insulin pump 23, respectively, and configured to supply power to these components. The power management unit 25 may operate in a rechargeable mode and may be equipped with overcharge and over-discharge protection circuits, which will not be elaborated here. The transmitter 22 may be connected to the control unit 21 and incorporated with a dual-working electrode sensor capable of collecting two channels of subcutaneous current signals related to blood glucose concentration, which are then transmitted to the control unit 21. The temperature sensor 24 may be integrated into the control unit 21 and configured to collect a body temperature of the target object. The control unit 21 may be connected to the insulin pump 23 and have a target model embedded therein. The control unit 21 is capable of processing the current signals to obtain the blood glucose concentration of the target object, calculating a minimum difference between a peak value of the blood glucose concentration and a normal range as a blood glucose concentration difference. Then, the control unit 21 acquires first monitoring data of the target object (here, the first monitoring data includes the blood glucose concentration difference). The first monitoring data is input into the target model to obtain an injection dosage, which is then transmitted to the insulin pump 23. Specifically, the control unit 21 is capable of data storage, computation, and control of other modules. The insulin pump 23 may include an infusion tube and an injection device. Under the control of the control unit 21, insulin is delivered to the injection device, then the injection device performs injection for the target object according to instructions from the control unit 21 (the instructions are related to the injection dosage).

Furthermore, in addition to the blood glucose concentration difference, the first monitoring data may further include the body temperature. The body temperature is used to perform temperature correction on the blood glucose concentration to accurately determine the blood glucose concentration. A similar approach applies to second monitoring data, which will not be redundantly explained here. It should be noted that when the first monitoring data does not include the body temperature, a dataset of the target model also excludes the body temperature.

Exemplarily, after the target object takes food, the blood glucose concentration will rise rapidly, and the first monitoring data including the body temperature and blood glucose concentration is collected. When the blood glucose concentration reaches the peak value, insulin intervention is required to lower blood glucose. The minimum difference between the peak value of the blood glucose concentration and the normal range is calculated to obtain the blood glucose concentration difference. The blood glucose concentration difference and the temperature are transmitted as input to the target model to calculate the injection dosage of insulin. Subsequently, an infusion system operates and drives the motor to inject insulin. Second monitoring data of the target object is monitored continuously. When the blood glucose concentration in the second monitoring data is not within a preset threshold range of a target blood glucose concentration, a protection mechanism may be triggered to adjust a redelivery dosage. The protection mechanism remains active until the blood glucose concentration is restored to a normal range. Concurrently, a total dosage of a prediction failure may be acquired and fed back to the target model. This feedback is configured to update weights of base models, specifically by adjusting the weights of a first group of base models identified based on the prediction failure, thereby enhancing the predictive capability of the target model.

In other embodiments, the embodiments of the related insulin pump control method may be executed through the cooperation among the components in the lower-level computer, which is not repeated here.

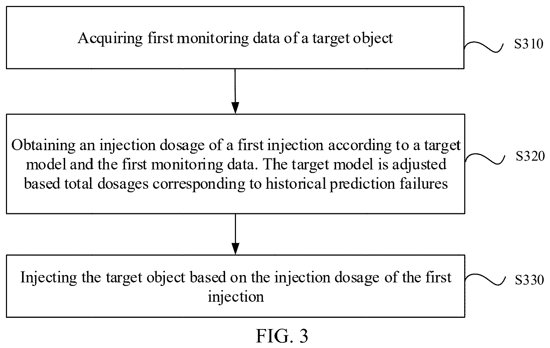

FIG. 3 is a flowchart of an insulin pump control method in an embodiment of the present invention. Referring to FIG. 3, the process includes step 310 to step 330.

Step 310 includes: acquiring first monitoring data of a target object.

Step 320 includes: obtaining an injection dosage of a first injection according to a target model and the first monitoring data. The target model is adjusted based total dosages corresponding to historical prediction failures.

Step 330 includes: injecting the target object based on the injection dosage of the first injection.

Specifically, this embodiment may be implemented in a lower-level computer. During use, current signals of the target object may be acquired by using a transmitter in the lower-level computer. The blood glucose concentration may be calculated according to the current signals, and a minimum difference between the peak value of the blood glucose concentration and the normal range may be calculated, thereby obtaining a blood glucose concentration difference. In this way, the first monitoring data (including the blood glucose concentration difference) of the target object may be acquired. The first monitoring data may be input into the target model adjusted based on the total dosages corresponding to historical prediction failures to obtain a quantified injection dosage of the first injection. Finally, under control of a control unit, insulin is delivered to an injection device of the insulin pump, and the injection device injects the target object according to an instruction (the instruction is related to the injection dosage of the first injection) of the control unit. The injection in the present invention is intended to assist in monitoring data, and is not intended to be a treatment.

The target model is adjusted based on the total dosages corresponding to historical prediction failures. The total dosages corresponding to historical prediction failures are defined as the cumulative insulin dosage delivered during a preceding injection cycle to restore the blood glucose concentration to within a target threshold, which equals the sum of the initially predicted dosage and any subsequent supplementary dosages administered by the protection mechanism.

In this embodiment, the first monitoring data of the target object is acquired, the injection dosage of the first injection is obtained according to the target model and the first monitoring data, the target model is adjusted based on the total dosages corresponding to historical prediction failures, and injection for the target object is performed based on the injection dosage of the first injection. Thus, the issues of inaccurate injection dosage and poor user experience with insulin pumps in the related art are solved. The target model adjusted based on the total dosages corresponding to historical prediction failures enables accurate estimation of the injection dosage for the target object, effectively mitigates the impact of individual physiological state variations and inter-individual differences on the target model, and improves prediction accuracy of the target model, thereby enhancing user experience.

In another embodiment, when the first monitoring data further includes the body temperature, the temperature sensor in the lower-level computer may be configured to acquire the body temperature of the target object, which will not be elaborated further here.

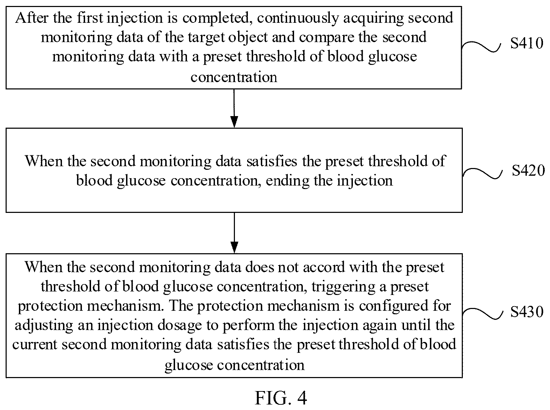

In some embodiments, referring to FIG. 4, the insulin pump control method may further include the following step 410 to step 430.

Step 410: after the first injection is completed, continuously acquiring second monitoring data of the target object and comparing the second monitoring data with a preset threshold of blood glucose concentration.

Step 420: when the second monitoring data satisfies the preset threshold of blood glucose concentration, ending the injection.

Step 430: when the second monitoring data does not accord with the preset threshold of blood glucose concentration, triggering a preset protection mechanism. The protection mechanism is configured for adjusting an injection dosage to perform the injection again until the second monitoring data satisfies the preset threshold of blood glucose concentration.

Specifically, the protection mechanism may be established to achieve full-process automation. The protection mechanism may involve adjusting an injection dosage to perform the injection again until the second monitoring data satisfies the preset threshold of blood glucose concentration. That is, after the first injection is completed, the target object may be continuously monitored at preset intervals to acquire the second monitoring data of the target object. The second monitoring data may be compared with the preset threshold of blood glucose concentration. When the second monitoring data falls within a preset threshold range of blood glucose concentration, the injection is ended. When the second monitoring data falls below a minimum value of the preset threshold range of blood glucose concentration or exceeds a maximum value of the preset threshold range of blood glucose concentration, the preset protection mechanism may be triggered to adjust the injection dosage to perform the injection again until the second monitoring data satisfies the preset threshold of blood glucose concentration. For example, when the second monitoring data falls below the minimum value of the preset threshold range of blood glucose concentration, an injection dosage required for a secondary injection to bring the second monitoring data accord with the preset threshold of blood glucose concentration is re-calculated. When the second monitoring data exceeds the maximum value of the preset threshold range of blood glucose concentration, the injection dosage required for the secondary injection to bring the second monitoring data accord with the preset threshold of blood glucose concentration is re-calculated.

According to this embodiment, the issues of inaccurate dosage caused by individual physiological state variations can be eliminated automatically, and thus the user experience is improved.

In some embodiments, the insulin pump control method may further include the following steps: acquiring a total dosage of a prediction failure, and adjusting weights of extreme learning machine base models in the target model based on the total dosage of the prediction failure. The weights determine how to fuse the individual predictions from the base models to produce the final dosage prediction.

Specifically, upon triggering the preset protection mechanism, it may indicate that the injection dosage predicted by the target model is insufficiently accurate. Consequently, the total dosage of the prediction failure may be obtained. The total dosage of the prediction failure may refer to the corresponding injection dosage required for the monitoring data to accord with the preset threshold of blood glucose concentration, which may be a cumulative injection dosage obtained by adding a supplementary injection dosage to the first injection dosage. The supplementary injection dosage may be the sum of two or more injection dosages. It should be noted that acquiring the total dosage of the prediction failure is not limited to the condition of triggering the preset protection mechanism.

Finally, the weights of the extreme learning machine base models in the target model may be adjusted based on the total dosage of the prediction failure. An adjustment method may include, but not be limited to, directly or indirectly improving the weights of the extreme learning machine base models matching the total dosage of predicted failure, and directly or indirectly decreasing the weights of the extreme learning machine base models not matching the total dosage of predicted failure, which is not limited.

According to the present embodiment, the target model can be rapidly adjusted to be adapted to individual physiological state variations by adjusting the weights of the extreme learning machine base models, eliminating the need to retrain the target model and thereby reducing hardware requirements for the lower-level computer.

It should be noted that the division into different groups of base models can be considered as being based on the total dosage of the prediction failure and according to different preset condition. Specifically, at least two groups of base models may be divided. The preset condition may include dosage matching.

In some embodiments, adjusting the weights of the extreme learning machine base models in the target model based on the total dosage of the current prediction failure, may include: determining a first group of base models based on the total dosage of the current prediction failure, improving weights of the first group of base models in the target model, and allocating remaining weights to non-first the second group of base models. The improved weights of the first group of base models may be higher than the allocated weights of the non-first second group of base models.

The case involving two groups of base models is explained below.

In some embodiments, adjusting the weights of the extreme learning machine base models in the target model based on the total dosage of the prediction failure, may include the following steps: determining a first group of base models and a second group of base models based on the total dosage of the prediction failure, improving weights of the first group of base models in the target model, and allocating remaining weights to the second group of base models. The first group of base models may include extreme learning machine base models matched with the total dosage of the prediction failure in the target model, and the second group of base models may include remaining extreme learning machine base models in the target model. The improved weights of the first group of base models may be higher than the allocated weights of the second group of base models.

In this embodiment, there are two groups of base models including a first group of base models and a second group of base models. The number of extreme learning machine base models in the first group of base models is at least one. For example, when a first condition is minimum prediction error, the extreme learning machine base models with the minimum prediction error may be divided into the first group of base models, and when a second condition is other than the minimum prediction error, remaining extreme learning machine base models other than the ones with the minimum prediction error may form the second group of base models. Exemplarily, there are 4 extreme learning machine base models including an extreme learning machine base model G1 with a predicted dosage (characteristic value) of 8; an extreme learning machine base model G2 with a predicted dosage (characteristic value) of 10; an extreme learning machine base model G3 with a predicted dosage (characteristic value) of 11; and an extreme learning machine base model G4 with a predicted dosage (characteristic value) of 12. When the total dosage of the prediction failure is 10.1, the first group of base models may include G2, and the second group of base models may include G1, G3, and G4. When the total dosage of the prediction failure is 9, the first group of base models may include G2 and G1, and the second group of base models may include G3 and G4. Alternatively, the first condition may define an extreme learning machine base model with the minimum prediction error. In cases where a plurality of base models satisfy the first condition for the first group, one of the plurality of base models may be randomly selected as the first group of base models, and the rest of the plurality of base models may be assigned to the second group. This approach may help reduce computational overhead.

For another example, when the first condition is that the prediction error satisfies a certain prediction error threshold, the extreme learning machine base models whose prediction errors satisfy the prediction error threshold may be divided into the first group of base models, and when the second condition is other than that the prediction error satisfies the certain prediction error threshold, remaining extreme learning machine base models other than the ones whose prediction errors satisfy the prediction threshold may form the second group of base models. When the total dosage of the prediction failure is 10 and the prediction error threshold is 1, the first group of base models may include G2 and G3, and the second group of base models may include G1 and G4.

After the first group of base models and the second group of base models are determined, weights of the first group of base models in the target model may be increased, and remaining weights are allocated to the second group of base models. At this point, the increased weights of the first group of base models may be higher than the weights allocated to the second group of base models. For example, the weights of the first group of base models in the target model may be increased, and the remaining weights may be evenly allocated to the second group of base models. The specific weight adjustment may be expressed as follows:

$$L_{\text{test\_new}} = G_\alpha \frac{E_{max}}{\sum_{i=1}^{T} E_i} + G_\beta \left(1 - \frac{E_{max}}{\sum_{i=1}^{T} E_i}\right);$$

$G_\alpha$ represents an extreme learning machine base model with the minimum prediction error, i.e., the first group of base models, $G_\beta$ represents the remaining extreme learning machine base models, i.e., a second group of base models, $E_i$ represents a sample error of an i-th base model, $T$ represents the quantity of base models, $E_{max}$ represents the largest sample error of the base model. Then, the updated target model is: $y_{p\_new} = G(L_{\text{test\_new}})$.

For another example, the weights of the first group of base models in the target model may be increased, and remaining weights may be randomly allocated to the second group of base models. In other embodiments, the specific allocation method is not limited.

According to the present embodiment, the first group of base models and the second group of base models may be determined based on the total dosage of the prediction failure. The weights of the first group of base models in the target model may be increased, and the remaining weights may be allocated to the second group of base models. The entire process may require no preset parameters and eliminate the need for user calibration, achieving a self-adaptive adjustment effect.

The case involving three or more groups of base models is explained below.

Specifically, the grouped base models may include a first group of base models, a second group of base models, ..., and an N-th group of base models. The base models satisfying the first condition form the first group of base models, the base models satisfying the second condition form the second group of base models, and the base models satisfying an N-th condition form the N-th group of base models. For example, the first condition may be that the difference (prediction error) from the total dosage of the prediction failure is less than a first threshold, the second condition is that the difference (prediction error) from the total dosage of the prediction failure is greater than the first threshold and less than a second threshold, and the N-th condition is that the difference (prediction error) from the total dosage of the prediction failure is greater than an (N-1)-th threshold and less than an N-th threshold.

After various groups of base models are determined, the weights of the first group of base models in the target model may be increased, and the remaining weights may be allocated to the other groups of base models. In this case, the increased weights of the first group of base models may be higher than the weights allocated to the other groups of base models. The remaining weights may be evenly allocated among the other groups of base models, or, the weights may be allocated by redefining proportions based on the prediction errors. Alternatively, the weights of some groups among the remaining groups of base models may remain unchanged while others may be reduced. The specific allocation method is not limited.

According to the present embodiment, a plurality of groups of base models may be determined based on the total dosage of the prediction failure, the weights of the first group of base models in the target model may be increased, and the remaining weights may be allocated to the remaining groups of base models. The entire process may require no preset parameters and eliminate the need for user calibration, achieving a self-adaptive adjustment effect.

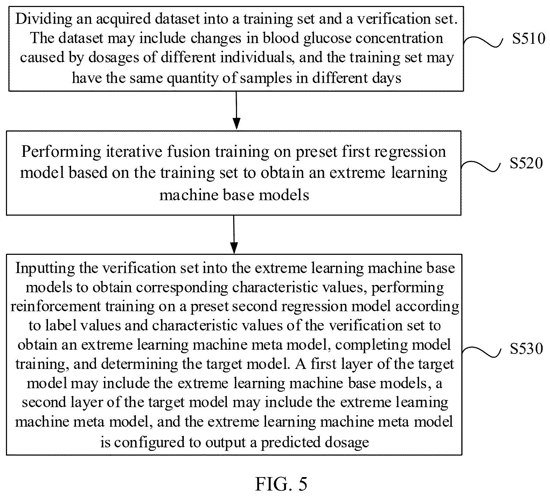

In some embodiments, referring to FIG. 5, the insulin pump control method may further include the following step: pre-training the target model. Pre-training of the target model may specifically include step 510 to step 530.

Step 510 may include dividing an acquired dataset into a training set and a verification set. The dataset may include changes in blood glucose concentration caused by dosages of different individuals, and the training set may have the same quantity of samples in different days.

Step 520 may include performing iterative fusion training on preset first regression model based on the training set to obtain extreme learning machine base models.

Step 530 may include inputting the verification set into the extreme learning machine base models to obtain corresponding characteristic values, performing reinforcement training on a preset second regression model according to label values and characteristic values of the verification set to obtain an extreme learning machine meta model, completing model training, and determining the target model. A first layer of the target model may include the extreme learning machine base models, a second layer of the target model may include the extreme learning machine meta model, and the extreme learning machine meta model is configured to output a predicted dosage.

Specifically, in practical applications, a method for acquiring the training set in the embodiments of the present invention may include, but not be limited to, acquiring a training set that satisfies the aforementioned requirements and is pre-stored in a database, downloading a qualified training set from an online platform, or generating a corresponding training set based on specific requirements. The embodiments of the present invention impose no limitations on the method for acquiring the training set. The training set may include datasets including blood glucose concentration variations caused by dosages of different individuals. Specifically, blood glucose concentration variations caused by dosages of different individuals in consecutive or at intervals over several days may be acquired. In addition, the training set may have the same quantity of samples in different days, thereby mitigating the impact of individual physiological states on model training. The dataset may be divided into the training set and the verification set by methods such as K-fold cross-verification, one-left method, or equal-proportion hierarchical sampling. In this embodiment, no restrictions are imposed on the division method of the training set and the verification set. The dataset may include textual data.

The training process of the target model may be considered as the training of the extreme learning machine base models (the first layer) and the extreme learning machine meta model (the second layer) in the target model. Both the first regression model and the second regression model may adopt a network model structure of a single hidden layer feedforward neural network (SLFN), which includes an input layer, a hidden layer, and an output layer. The training of the first layer may include performing iterative fusion training on the preset first regression model based on the training set to obtain the extreme learning machine base models. The adopted iterative fusion training may be considered as follows: random input layer weights and biases (for each of extreme learning machine base models in the first layer) are adopted, while output layer weights (for each of extreme learning machine base models in the first layer) are calculated through a generalized inverse matrix theory. Once the weights and biases on all network nodes are obtained, the training of the extreme learning machine base models is completed. It should be noted that one extreme learning machine base model may be added in each iteration.

After the first layer is trained, the verification set may be input into the extreme learning machine base models in the first layer to obtain corresponding characteristic values, the reinforcement training may be performed on the preset second regression model according to label values and the characteristic values of the verification set, thereby obtaining the extreme learning machine meta model. The extreme learning machine meta model is capable of outputting the predicted dosage. The process of reinforcement training may be similar to the process of fusion training but lack the iterative steps, which is not repeated. At this point, the overall training may be considered complete, and the target model may be obtained. Furthermore, the reinforcement learning approach may help mitigate the impact of individual differences on model training.

It should be noted that when the monitoring data of the target object is input into the target model, the monitoring data may sequentially pass through the two layers, and the final prediction may be obtained, which corresponds to the injection dosage for the target object. After the injection dosage is obtained, information processing or analysis may be further performed according to the injection dosage, and the processed or analyzed results may subsequently be applied in various scenarios.

In the related art, linear regression modeling methods may commonly be used to construct dosage models, which are then employed to output insulin dosages. However, due to individual differences and physiological states, the injection dosages of the insulin pump derived from linear regression modeling methods may often be inaccurate, leading to poor user experience. In contrast, in the embodiment of the present invention, the acquired dataset may be divided into the training set and the verification set. The dataset may include blood glucose concentration variations and body temperatures caused by dosages of different individuals. The training set may have the same quantity of samples in different days, thereby mitigating the impact of individual physiological state variations on model training. Based on the training set, iterative fusion training may be performed on the preset first regression model to obtain the extreme learning machine base models. The verification set may be input into the extreme learning machine base models to obtain corresponding characteristic values. According to the label values and the characteristic values of the verification set, the reinforcement training may be performed on the preset second regression model to obtain an extreme learning machine meta model. This reinforcement learning approach may mitigate the impact of individual differences on model training, thereby completing the model training and determining the target model. The first layer of the target model may include the extreme learning machine base models, and the second layer of the target model may include the extreme learning machine meta model. The extreme learning machine meta model may be configured for outputting predicted dosages. This approach solves the issue of inaccurate predictions of dosage models in related art, which fail to satisfy practical requirements. By controlling the quantity of samples across different days in the training set and combining the training of the two layers of regression models, the prediction accuracy of the target model may be significantly enhanced.

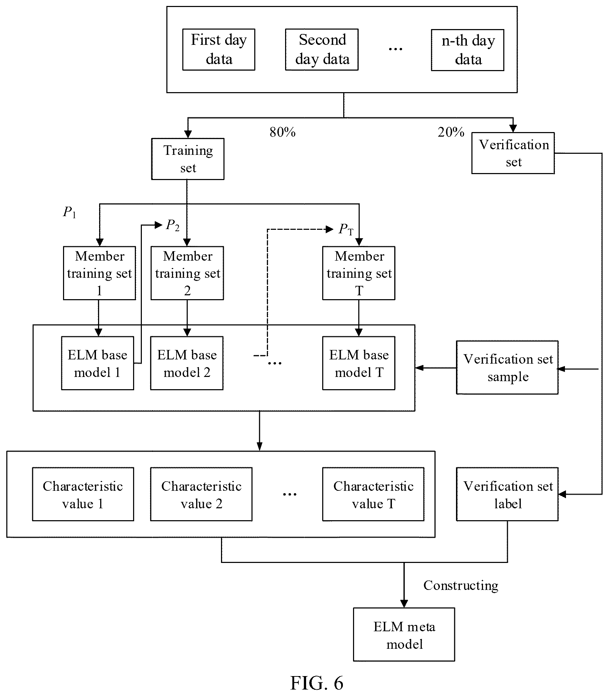

The foregoing steps are described in detail below with reference to FIG. 6.

Furthermore, the dataset may further include the body temperature in addition to the blood glucose concentration difference. The temperature correction may be performed on the blood glucose concentration by using the body temperature to acquire the blood glucose concentration accurately. Accordingly, the divided training set and the verification set may also include the body temperature. The training on the target model using different datasets may be similar and not be redundantly explained here.

In some embodiments, dividing the acquired dataset into the training set and the verification set in step 510 may include the following steps: acquiring blood glucose concentration changes caused by dosages of different individuals as the dataset, and dividing the dataset into the training set and the verification set by equal-proportion hierarchical sampling.

Specifically, blood glucose concentration changes caused by dosages of different individuals may be obtained as the dataset through means such as database or network platforms. The equal-proportion hierarchical sampling may be applied to divide the dataset into the training set and the verification set, thereby ensuring to the greatest extent that the same quantity of samples across different days in the training set remain consistent, so as to mitigate the impact of individual physiological states on model training.

The equal-proportion hierarchical sampling may involve selecting a corresponding quantity of samples according to the proportion of each layer in the overall when selecting samples in each layer. The obtained training set may be represented as $\{(X_i, Y_i), i=1, \cdots, n\}$, $X_i$ may represent a training set sample, and Y may represent a training set label. The obtained verification set may be represented as $\{(X_j, Y_j), j = 1, \cdots, m\}$, $X_j$ may represent a verification set sample and $Y_j$ may represent a verification set label. Furthermore, the training set may account for 80% of the total dataset, and the verification set may account for 20%. $n, m$ may represent the quantity of samples in the training set and the verification set, respectively.

In some embodiments, performing the iterative fusion training on the preset first regression model based on the training set to obtain the extreme learning machine base models in step 520 may include the following steps: initializing weights of the samples in the training set based on preset number of iterations, and determining a sampling probability of the samples; selecting first training samples for a iteration round from the training set based on the sampling probability; performing fusion training on the preset first regression model based on the first training samples to obtain an extreme learning machine base model for the iteration round; and when error rates corresponding to the characteristic values obtained in all iteration rounds meet a termination condition, obtaining all the extreme learning machine base models.

Specifically, the number of iterations may be preset based on an extreme learning machine (ELM) algorithm. The ELM algorithm is a single-hidden-layer feedforward neural network including an input layer, a hidden layer, and an output layer. The hidden layer is located in the middle, fully connected between the input layer and the output layer. The algorithm operates as follows.

It may be assumed that a sample $(X_i, Y_i)$ is input to the input layer, $X_i = [x_{i,1}, x_{i,2}, \cdots, x_{i,n}]^T \in R^n$, and $Y_i = [y_{i,1}, y_{i,2}, \cdots, y_{i,n}]^T \in R^m$. X, represents a training set sample, and Y represents a training set label.

An output function of the hidden layer may be expressed as: $f_L(x) = \sum_{i=1}^{L} \beta_i G(w_i \cdot x_i + b_i)$. $L$ may represent the number of nodes in the hidden layer, $G(x)$ may represent an activation function, $w_i = [w_{i,1}, w_{i,2}, \cdots, w_{i,n}]^T$ may represent an input weight vector; $\beta_i$ may represent an output weight coefficient, $b_i$ may represent a bias coefficient of the i-th hidden unit, and $f_L(x)$ may represent an output function of the hidden layer.

The learning objective is to minimize $\|H\beta - Y\|^2$, so that it infinitely approaches 0.

The matrix representation is expressed as: $H\beta = Y$,

$$ H = \begin{bmatrix} h(x_1) \\ \vdots \\ h(x_N) \end{bmatrix} = \begin{bmatrix} G(\omega_1^T \cdot x_1 + b_1) & \cdots & G(\omega_L^T \cdot x_1 + b_L) \\ \vdots & \cdots & \vdots \\ G(\omega_1^T \cdot x_N + b_1) & \cdots & G(\omega_L^T \cdot x_N + b_L) \end{bmatrix}_{N \times L}, \beta = \begin{bmatrix} \beta_1^T \\ \vdots \\ \beta_L^T \end{bmatrix}, Y = \begin{bmatrix} y_1^T \\ \vdots \\ y_L^T \end{bmatrix}. $$

Then, the solution for $\beta$ can be derived as: $\beta = H^+ \times Y$, $H^+$ may represent a generalized inverse of the matrix $H$. It may be considered that the extreme learning machine takes the following as input: the sample $(X_i, Y_i)$, the number $L$ of nodes in the hidden layer, and the activation function $G(x)$; and the extreme learning machine takes the following as output: the output weight coefficient $\beta_i$, the bias coefficients $b$ of the hidden units, and the output function $f_L(x)$ of the hidden layer.

Then, the process of iterative fusion training based on the above extreme learning machine may be as follows.

The number of iterations may be set as $t = 1, 2, \cdots, T$, the sample weights may be initialized as $W_i^{(1)} = 1/n$, $i = 1, 2, \cdots, n$, and n may represent the quantity of samples. Then the following steps may be performed.

1. Calculating a sampling probability for each sample in an original training set as: $P_j^{(t)} = W_j^{(t)} / \sum_{j=1}^{n} W_j^{(t)}$.

2. Selecting new training samples for a t-th round from the original training set based on the sampling probability as the first training samples through a roulette wheel selection method, a member of the first regression model $G_t(X)$ may be established by adopting the ELM algorithm, and the first training samples may be input into the model to obtain the characteristic values of all the first training samples, which are expressed as: $\hat{Y}_i^{(t)}$, $i = 1, 2, \cdots, n$.

3. Calculating an error for each sample in a first training set, expressed as:

$$E_i^{(t)} = \frac{\left| \hat{Y}_i^{(t)} - Y_i \right|}{\max \left| \hat{Y}_i^{(t)} - Y_i \right|}, i = 1, 2, \cdots, n.$$

4. Calculating a weighted error sum for the t-th round, expressed as: $\bar{E}_t = \sum_{i=1}^{n} E_i P_i^{(t)}$ .

5. Calculating a commonality index, expressed as: $\beta_t = \dfrac{\bar{E}_t}{1 - \bar{E}_t}$ .

6. Updating the weight of each sample in the first training set, expressed as:

$$W_i^{(t+1)} = W_i^{(t)} \beta_t^{[1 - E_i^{(t)}]}.$$

After repeating steps 1 to 6, in each iteration process, a first regression model may be constructed and the error thereof may be calculated. The sample weights may be updated according to the error, improving the sampling probability of poor samples, and a new training set may be generated for the fusion training of a next first regression model. The cycle may continue until the total prediction error satisfies the ending condition. That is, each iteration of the fusion training may construct a first regression model, and after training is completed, a corresponding extreme learning machine base model (ELM base model) may be obtained.

According to the embodiment, iterative fusion training can be efficiently completed to obtain the extreme learning machine base models, reducing training time while ensuring training accuracy.

In some embodiments, in order to reduce the number of extreme learning machine base models and accelerate a convergence speed of the first layer training as well as overall computational efficiency, the optimal number of iterations may be obtained by using an iteration formula.

The iterative formula may be expressed as: $(\sum_{t=1}^{t_b} \log \frac{1}{\beta_t}) \geq (\frac{1}{2} \sum_{t=1}^{T} \log \frac{1}{\beta_t})$ . $t_b$ may represent the optimal number of iterations, and $\beta_t$ may represent the commonality index.

In some embodiments, performing reinforcement training on the preset second regression model according to label values and characteristic values of the verification set to obtain the extreme learning machine meta model in step 530 may include the following steps: constructing second training samples for the preset second regression model according to the label values and the characteristic values of the verification set. and performing the reinforcement training on the second regression model based on the second training samples to obtain the extreme learning machine meta model.

Specifically, for a verification set $\{(X_j, Y_j), j = 1, \cdots, m\}$, a matrix $L$ of a set of characteristic values of the verification set may be obtained through calculation by using $T$ extreme learning machine base models:

$$L = \begin{bmatrix} G_1(x_1) & G_2(x_1) & \cdots & G_T(x_1) \\ G_1(x_2) & G_2(x_2) & \cdots & G_T(x_2) \\ \vdots & \vdots & \cdots & \vdots \\ G_1(x_m) & G_2(x_m) & \cdots & G_T(x_m) \end{bmatrix}$$

The label values of the verification set may be organized into a matrix Y, represented as $Y = [y_1, y_2, \cdots, y_m]$. The combination of the matrix Y and the matrix L may form a set $\{(L_{m \times T}, Y_{m \times 1})\}$ of second training samples of the second regression model. Based on the second training samples, the reinforcement training may be performed on the second regression model to obtain an extreme learning machine meta model (ELM meta model) $G(L)$. The reinforcement training process may also be implemented by using the ELM algorithm, which is not repeated here.

According to the embodiment, the reinforcement training can be efficiently completed to obtain the extreme learning machine meta model, reducing training time while ensuring training accuracy.

It should be noted that, when the target model is applied to the lower-level computer, and the first monitoring data is taken as an unknown sample to input to the target model, a matrix $L_{test} = [G_1(x), G_2(x), \cdots, G_T(x)]$ of characteristic values may be obtained through $T$ extreme learning machine base models in the first layer. Then, the injection dosage may be predicted and output through the extreme learning machine meta model in the second layer. The injection dosage may be represented as:

$$y_p = G(L_{test}).$$

In some embodiments, another insulin pump control method is provided, including: acquiring first monitoring data of a target object, inputting the first monitoring data into a trained extreme learning machine model to determine an injection dosage, and injecting the target object based on the injection dosage. The first monitoring data includes blood glucose data.

In some embodiments, the trained extreme learning machine model includes an extreme learning machine base model and an extreme learning machine meta model, an input of the extreme learning machine base model includes the first monitoring data, an output of the extreme learning machine base model is taken as an input of the extreme learning machine meta model, and an output of the extreme learning machine meta model includes the injection dosage.

In some embodiments, the method further includes: after the injection is completed, continuously acquiring second monitoring data of the target object, and comparing the second monitoring data with a preset threshold of blood glucose concentration, and when the second monitoring data does not accord with the preset threshold of blood glucose concentration, performing injection again according to a preset dosage.

In some embodiments, the method further includes: determining, based on a combination of the injection dosage and the reinjection dosage, whether a difference between the output of the extreme learning machine base model and the combination meets a preset condition; if the difference between the output of the extreme learning machine base model and the combination meets the preset condition, adjusting a weight of the extreme learning machine base model in the trained extreme learning machine model. Furthermore, the preset condition includes the difference is greater than a prediction error threshold.

It should be noted that the steps illustrated in the above process or the flowcharts of the drawings may be executed in a computer system, such as a set of computer-executable instructions. Although a logical order is shown in the flowcharts, in some cases, the steps illustrated or described may be executed in an order different from that presented herein.

An insulin pump control apparatus is further provided in an embodiment of the present invention, which is configured to implement the above embodiments. Details already explained will not be repeated below. Terms such as "module", "unit", and "sub-unit" used herein may refer to a combination of software and/or hardware capable of achieving preset functions. Although the apparatus described in the following embodiments may be implemented in software, implementation via hardware, or a combination of software and hardware, may also be possible and contemplated.

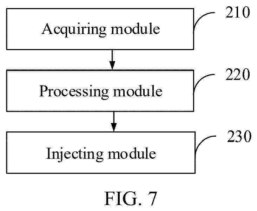

FIG. 7 is a structural block diagram of an insulin pump control apparatus in this embodiment. Referring to FIG. 7, the apparatus may include an acquisition module 210, a processing module 220, and an injecting module 230.

The acquisition module 210 may be configured to acquire first monitoring data of a target object.

The processing module 220 may be configured to obtain an injection dosage of a first injection according to a target model and the first monitoring data. The target model is adjusted based on total dosages corresponding to historical prediction failures.

The injection module 230 may be configured to inject the target object based on the injection dosage.

Through the apparatus, the issues of inaccurate injection dosage and poor user experience with insulin pumps in the related art are solved. By utilizing the built-in target model to accurately predict the injection dosage for the target object, user experience is enhanced. Furthermore, through the processing of database in the target model, the impact of individual physiological state variations and inter-individual differences on the target model is effectively mitigated, thereby improving the prediction accuracy of the target model.

In some embodiments, the insulin pump control apparatus may further include a protection module.

The protection module may be configured to, after the first injection is completed, continuously acquire second monitoring data of the target object, and compare the second monitoring data with a preset threshold of blood glucose concentration; end the injection when the second monitoring data satisfies the preset threshold of blood glucose concentration; and trigger a preset protection mechanism when the second monitoring data does not accord with the preset threshold of blood glucose concentration. The protection mechanism may be configured for adjusting an injection dosage to perform the injection again until the second monitoring data satisfies the preset threshold of blood glucose concentration.

In some embodiments, the insulin pump control apparatus may further include an adjustment module.

The adjustment module may be configured to, after triggering the preset protection mechanism, acquire a total dosage of a prediction failure; and adjust weights of extreme learning machine base models in the target model based on the total dosage of the prediction failure.

In some embodiments, the adjustment module may be further configured to determine a first group of base models and a second group of base models based on the total dosage of the prediction failure. The first group of base models may include extreme learning machine base models matched with the total dosage of the prediction failure in the target model, and the second group of base models may include remaining extreme learning machine base models in the target model. The adjustment module may be further configured to improve weights of the first group of base models in the target model, and allocating remaining weights to the second group of base models. The improved weights of the first group of base models may be higher than the allocated weights of the second group of base models.

In some embodiments, the insulin pump control apparatus may further include a pre-training module.

The pre-training module may be configured to pre-train the target model, which may specifically include: divide an acquired dataset into a training set and a verification set, perform iterative fusion training on a preset first regression model based on the training set to obtain extreme learning machine base models, input the verification set into the extreme learning machine base models to obtain corresponding characteristic values, and perform reinforcement training on a preset second regression model according to label values and the characteristic values of the verification set to obtain an extreme learning machine meta model, completing model training, and determining the target model. The dataset may include changes in blood glucose concentration caused by dosages of different individuals, and the training set may have the same quantity of samples in different days. A first layer of the target model may include the extreme learning machine base model, and a second layer of the target model may include the extreme learning machine meta model. The extreme learning machine meta model may be configured to output a predicted dosage.

In some embodiments, the pre-training module may be further configured to initialize weights of the samples in the training set based on preset number of iterations, and determine a sampling probability of the samples; select first training samples for a iteration round from the training set based on the sampling probability; perform fusion training on the preset first regression model based on the first training samples to obtain an extreme learning machine base model for the iteration round; and when error rates corresponding to the characteristic values obtained in all iteration rounds meet a termination condition, obtain all the extreme learning machine base models.

In some embodiments, the pre-training module may be further configured to construct second training samples for the preset second regression model according to the label values and the characteristic values of the verification set, and perform the reinforcement training on the second regression model based on the second training samples to obtain the extreme learning machine meta model.

It should be noted that the above modules may be functional modules or program modules, and may be implemented via software or hardware. For modules implemented via hardware, the modules may be located in a same processor. Alternatively, the modules may be distributed across different processors in any arbitrary combination.

In addition, in conjunction with the insulin pump control method provided in the above embodiments, a storage medium may be further provided in this embodiment for implementation. The storage medium has a computer program stored thereon. When the computer program is executed by the processor, any insulin pump control method in the above embodiments is implemented.

It should be noted that the information and data involved in the present invention are authorized by users or fully authorized by relevant parties, and are legally used.

It should be appreciated that the specific embodiments described herein are merely illustrative of this application and are not intended to be limiting. According to the embodiments provided in the present invention, all other embodiments obtained by those of skilled in the art without creative efforts shall fall within the protection scope of the present invention.

The accompanying drawings are only some examples or embodiments of the present invention, and those skilled in the art may also adapt the present invention to other similar scenarios according to the drawings without creative effort. In addition, it should be understood that although the work involved in the development process may be complex and lengthy, for those skilled in the art, some changes such as design, manufacturing or production performed according to the technical content disclosed in the present invention are merely conventional technical means, and should not be considered as indicating insufficient disclosure in the present invention.

The term "embodiment" as used in the present invention means that specific features, structures, or characteristics described in connection with the embodiment may be included in at least one embodiment of the present invention. The appearance of this phrase at various locations in the description does not necessarily refer to the same embodiment, nor does it imply that embodiments are mutually exclusive, independent, or alternatives. Those skilled in the art can explicitly or implicitly understand that the embodiments described in the present invention may be combined with other embodiments without conflict.

The above embodiments only express several implementations of the present invention. The description thereof is specific and detailed, but should not be construed as limiting the protection scope of the patent. It should be noted that, for those skilled in the art, several modifications and improvements may be made without departing from the concept of the present invention, which all fall within the protection scope of the present invention. Therefore, the protection scope of the present invention shall be defined by the appended claims.

**Claims**

1. An insulin pump control method, **characterized by** comprising:

acquiring first monitoring data of a target object;
obtaining an injection dosage of a first injection according to a target model and the first monitoring data, wherein the target model is adjusted based on total dosages corresponding to historical prediction failures; and
injecting the target object based on the injection dosage of the first injection.

2. The method of claim 1, further comprising:
after the first injection is completed, continuously acquiring second monitoring data of the target object, and comparing the second monitoring data with a preset threshold of blood glucose concentration,

when the second monitoring data satisfies the preset threshold of blood glucose concentration, ending the injection; and
when the second monitoring data does not accord with the preset threshold of blood glucose concentration, triggering a preset protection mechanism, wherein the protection mechanism is configured for adjusting an injection dosage to perform the injection again until the second monitoring data satisfies the preset threshold of blood glucose concentration.

3. The method of claim 1 or claim 2, further comprising:

acquiring a total dosage of a prediction failure; and
adjusting weights of the extreme learning machine base models in the target model based on the total dosage of the prediction failure.

4. The method of claim 3, wherein adjusting the weights of the extreme learning machine base models in the target model based on the total dosage of the prediction failure, comprises:

determining a first group of base models based on the total dosage of the prediction failure; and
improving weights of the first group of base models in the target model, and allocating remaining weights to non-first group of base models, wherein the improved weights of the first group of base models are higher than the allocated weights of the non-first group of base models.

5. The method of claim 4, wherein determining the first group of base models based on the total dosage of the prediction failure, comprises:
when a prediction error between a predicted dosage of an extreme learning machine base model in the target model and the total dosage of the prediction failure meets a preset condition, determining that the extreme learning machine base model belongs to the first group of base models, wherein the preset condition comprises: the prediction error is less than a preset prediction error threshold; or the prediction error is less than an error between a predicted dosage of other extreme learning machine base model and the total dosage of the prediction failure.

6. The method of any one of claims 1 to 5, further comprising:
pre-training the target model, wherein pre-training the target model comprises:

dividing an acquired dataset into a training set and a verification set, wherein the dataset comprises changes in blood glucose concentration caused by dosages of different individuals, and the training set has the same quantity of samples in different days;
performing iterative fusion training on a preset first regression model based on the training set to obtain extreme learning machine base models;
inputting the verification set into the extreme learning machine base models to obtain corresponding characteristic values; and
performing reinforcement training on a preset second regression model according to label values and the characteristic values of the verification set to obtain an extreme learning machine meta model, completing model training, and determining the target model, wherein a first layer of the target model comprises the extreme learning machine base models, a second layer of the target model comprises the extreme learning machine meta model, and the extreme learning machine meta model is configured to output a predicted dosage.

7. The method of claim 6, wherein performing the iterative fusion training on the preset first regression model based on the training set to obtain the extreme learning machine base models, comprises:

   initializing weights of the samples in the training set based on preset number of iterations, and determining a sampling probability of the samples;
   selecting first training samples for an iteration round from the training set based on the sampling probability;
   performing fusion training on the preset first regression model based on the first training samples to obtain an extreme learning machine base model for the iteration round; and
   when error rates corresponding to the characteristic values obtained in all iteration rounds meet a termination condition, obtaining all the extreme learning machine base models.

8. The method of claim 6 or claim 7, wherein performing the reinforcement training on the preset second regression model according to the label values and the characteristic values of the verification set to obtain the extreme learning machine meta model, comprises:

   constructing second training samples for the preset second regression model according to the label values and the characteristic values of the verification set; and
   performing the reinforcement training on the second regression model based on the second training samples to obtain the extreme learning machine meta model.

9. An insulin pump control method, comprising:

   acquiring first monitoring data of a target object, wherein the first monitoring data comprises blood glucose data;
   inputting the first monitoring data into a trained extreme learning machine model to determine an injection dosage; and
   injecting the target object based on the injection dosage.

10. The method of claim 9, wherein the trained extreme learning machine model comprises an extreme learning machine base model and an extreme learning machine meta model, an input of the extreme learning machine base model comprises the first monitoring data, an output of the extreme learning machine base model is taken as an input of the extreme learning machine meta model, and an output of the extreme learning machine meta model comprises the injection dosage.

11. The method of claim 10, further comprising:

   after the injection is completed, continuously acquiring second monitoring data of the target object, and comparing the second monitoring data with a preset threshold of blood glucose concentration; and
   when the second monitoring data does not accord with the preset threshold of blood glucose concentration, performing injection again according to a preset dosage.

12. The method of claim 11, further comprising:

   determining, based on a combination of the injection dosage and the reinjection dosage, whether a difference between the output of the extreme learning machine base model and the combination meets a preset condition;
   if the difference between the output of the extreme learning machine base model and the combination meets the preset condition, adjusting a weight of the extreme learning machine base model in the trained extreme learning machine model.

13. An insulin pump control apparatus, **characterized by** comprising an acquiring module (210), a processing module (220), and an injecting module (230),

   wherein the acquiring module (210) is configured for acquiring first monitoring data of a target object;
   the processing module (220) is configured for obtaining an injection dosage of a first injection according to a target model and the first monitoring data, wherein the target model is adjusted based on total dosages corresponding to historical prediction failures; and
   the injecting module (230) is configured for injecting the target object based on the injection dosage of the first injection.

**14.** An artificial pancreas closed-loop system, **characterized by** comprising a memory and a processor, wherein the memory has a computer program stored thereon, and the processor is configured to execute the computer program to implement the steps of the method of any one of claims 1 to 12.

**15.** A computer readable storage medium having a computer program stored thereon, **characterized in that** the computer program is executable by a processor to implement the steps of the method of any one of claims 1 to 12.

FIG. 1

FIG. 2

| Acquiring first monitoring data of a target object | S310 |

| Obtaining an injection dosage of a first injection according to a target model and the first monitoring data. The target model is adjusted based total dosages corresponding to historical prediction failures | S320 |

| Injecting the target object based on the injection dosage of the first injection | S330 |

FIG. 3

After the first injection is completed, continuously acquiring second monitoring data of the target object and compare the second monitoring data with a preset threshold of blood glucose concentration — S410

When the second monitoring data satisfies the preset threshold of blood glucose concentration, ending the injection — S420

When the second monitoring data does not accord with the preset threshold of blood glucose concentration, triggering a preset protection mechanism. The protection mechanism is configured for adjusting an injection dosage to perform the injection again until the current second monitoring data satisfies the preset threshold of blood glucose concentration — S430

FIG. 4

Dividing an acquired dataset into a training set and a verification set. The dataset may include changes in blood glucose concentration caused by dosages of different individuals, and the training set may have the same quantity of samples in different days ⟶ S510

Performing iterative fusion training on preset first regression model based on the training set to obtain an extreme learning machine base models ⟶ S520

Inputting the verification set into the extreme learning machine base models to obtain corresponding characteristic values, performing reinforcement training on a preset second regression model according to label values and characteristic values of the verification set to obtain an extreme learning machine meta model, completing model training, and determining the target model. A first layer of the target model may include the extreme learning machine base models, a second layer of the target model may include the extreme learning machine meta model, and the extreme learning machine meta model is configured to output a predicted dosage ⟶ S530

FIG. 5

FIG. 6

Acquiring module — 210

Processing module — 220

Injecting module — 230

FIG. 7

**EP 4 769 422 A1**

## EUROPEAN SEARCH REPORT

Application Number

EP 25 22 7427

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WOLDAREGAY ASHENAFI ZEBENE ET AL: "Data-driven modeling and prediction of blood glucose dynamics: Machine learning applications in type 1 diabetes", ARTIFICIAL INTELLIGENCE IN MEDICINE ELSEVIER, NL, vol. 98, 1 July 2019 (2019-07-01), pages 109-134, XP085817800, ISSN: 0933-3657, DOI: 10.1016/J.ARTMED.2019.07.007 [retrieved on 2019-07-26] * the whole document * | 1-15 | INV.<br>G16H20/17<br>A61M5/172<br>G16H50/20<br>G16H50/70 |
| X | Georga Eleni I. ET AL: "Online Prediction of Glucose Concentration in Type 1 Diabetes Using Extreme Learning Machines", 2015 37th Annual International Conference of the IEEE Engineering in Medicine and Biology Society (EMBC), 25 August 2015 (2015-08-25), pages 3262-3265, XP093387431, Milan, Italy Retrieved from the Internet: URL:https://ieeexplore.ieee.org/stamp/stamp.jsp?tp=&arnumber=7319088 * the whole document * | 1-15 | |
| | -----<br>-/-- | | TECHNICAL FIELDS SEARCHED (IPC)<br><br>G16H<br>A61M |

The present search report has been drawn up for all claims

| Place of search | Date of completion of the search | Examiner |
|---|---|---|
| Munich | 16 April 2026 | Eichenauer, Lars |

page 1 of 2

| | Europäisches Patentamt<br>European Patent Office<br>Office européen des brevets | **EUROPEAN SEARCH REPORT** | **Application Number**<br>EP 25 22 7427 |
| --- | --- | --- | --- |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | LING SAI HO ET AL: "Non-invasive hypoglycemia monitoring system using extreme learning machine for Type 1 diabetes",<br>ISA TRANSACTIONS. INSTRUMENT SOCIETY OF AMERICA. PITTSBURGH., US,<br>vol. 64, 13 June 2016 (2016-06-13), pages 440-446, XP029731728,<br>ISSN: 0019-0578, DOI:<br>10.1016/J.ISATRA.2016.05.008<br>* chapter "2.2 Extreme learning machine (ELM)-based neural network" *<br>----- | 1-15 | |
| X | ASSADI KHAOULA ET AL: "Estimation of blood glucose levels techniques",<br>2017 INTERNATIONAL CONFERENCE ON SMART, MONITORED AND CONTROLLED CITIES (SM2C) IEEE,<br>17 February 2017 (2017-02-17), pages 75-80, XP033226933,<br>DOI: 10.1109/SM2C.2017.8071822<br>[retrieved on 2017-10-18]<br>* the whole document *<br>----- | 1-15 | **TECHNICAL FIELDS SEARCHED (IPC)** |

The present search report has been drawn up for all claims

| Place of search | Date of completion of the search | Examiner |
| --- | --- | --- |
| Munich | 16 April 2026 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

page 2 of 2